# EUROPÄISCHE PATENTSCHRIFT

(11) **EP 3 095 779 B1**
(45) Veröffentlichungstag und Bekanntmachung des Hinweises auf die Patenterteilung: **04.04.2018**
(21) Anmeldenummer: 15168377.8
(22) Anmeldetag: 20.05.2015
(51) Int. Cl.: C07C 391/02

(54) **KUPPLUNG VON ZWEI ARENEN MIT SELENDIOXID ZU EINEM SELENOBIARYLETHER**
COUPLING OF TWO ARENES WITH SELENIUM DIOXIDE TO FORM A SELENOBIARYL ETHER
COUPLAGE DE DEUX ARÈNES DOTÉS D'ANHYDRIDE SÉLÉNIEUX À UN ÉTHER DE SÉLÉNIOBIARYL

(43) Veröffentlichungstag der Anmeldung: 23.11.2016
(73) Patentinhaber: Evonik Degussa GmbH, 45128 Essen (DE)
(72) Erfinder: DYBALLA, Katrin Marie, 45657 Recklinghausen (DE); FRANKE, Robert, 45772 Marl (DE); WALDVOGEL, Siegfried R., 55435 Gau-Algesheim (DE); QUELL, Thomas, 55118 Mainz (DE); MIRION, Michael, 55122 Mainz (DE)

(56) Entgegenhaltungen:
- TAPAN KANTI PAINE ET AL: "Manganese complexes of mixed O, X, O-donor ligands (X = S or Se): synthesis, characterization and catalytic reactivityElectronic supplementary information (ESI) available: Fig. S1 and S2, plots of the magnetic susceptibility measurements ? the experimental data with the simulations. See http://www.r", DALTON TRANSACTIONS, Nr. 15, 1. Januar 2003 (2003-01-01), Seiten 3136-3144, XP55215909, ISSN: 1477-9226, DOI: 10.1039/b304765m
- TAPAN K. PAINE ET AL: "Polynuclear Nickel(II) Complexes: Preparation and Magnetic Properties of NiII4, NiII5, and NiII6 Species with Ligands containing O[intersect]X[intersect]O (X = S, Se or N) Donor Atoms", EUROPEAN JOURNAL OF INORGANIC CHEMISTRY, Bd. 2003, Nr. 17, 1. September 2003 (2003-09-01), Seiten 3167-3178, XP55203672, ISSN: 1434-1948, DOI: 10.1002/ejic.200300164
- G. V. BOYD ET AL: "470. The action of selenious acid on alkyl ethers of phenols", JOURNAL OF THE CHEMICAL SOCIETY, 1. Januar 1949 (1949-01-01), Seiten 2196-2197, XP55223615, LETCHWORTH; GB ISSN: 0368-1769, DOI: 10.1039/jr9490002196

## Beschreibung

Die Erfindung betrifft ein Verfahren zur Kupplung von zwei Arenen mit Selendioxid zu einem Selenobiarylether.

Selenobiarylether stellen eine hochinteressante und vielversprechende Verbindungsklasse dar. Diese Verbindungen werden derzeit in bestimmte Komplexe, vor allem manganhaltig, eingebaut, besitzen aber ein großes Potential für weitere Anwendungen.

Der Begriff Arene wird in dieser Anmeldung als Gattungsbegriff verwendet und umfasst somit auch substituierte Arene, jedoch nicht Phenole.

T. K. Paine beschreibt eine Synthese von 2,2'-Selenobis(4,6-di-tert-butylphenol) unter Verwendung von Selendioxid. Die Herstellung von 2,2'-Selenobis(4,6-di-tert-butylphenol) erfolgt hier in einem sauren Medium unter Zusatz von konzentrierter Salzsäure. Das Produkt wird mit einer Ausbeute von 25 % erhalten (T. K. Paine et al., "Manganese complexes of mixed O, X, O-donor ligands (X = S or Se): synthesis, characterization and catalytic reactivity", Dalton Trans., 2003, 15, 3136-3144).
Hierbei ist besonders nachteilig, dass die Ausbeuten gering und somit verbesserungswürdig sind.
Weiterhin ist es problematisch, dass Salzsäure verwendet wird, da diese chlorhaltig ist. Chlorhaltige Spezies führen in stählernen Reaktoren zu Korrosionen, sodass bei großtechnischen Synthesen besondere Maßnahmen, die häufig mit großen Investitionen verbunden sind, ergriffen werden müssen (beispielsweise besondere Reaktormaterialien). Es ist daher wünschenswert das Verfahren zu verbessern.

H. M. Lin beschreibt eine Syntheseroute für Selenobiarylether, welche über mehrere Stufen erfolgt. Zuerst muss Brom an das entsprechende Phenol addiert werden, um das Produkt dann mit Magnesium zu einem Grignard-Reagenz umzusetzen. Das Grignard-Reagenz kann dann mit dem zugesetzten Selen reagieren, bevor die eigentliche Kupplung zum Biarylether erfolgt: 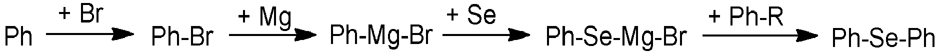 (H. M. Lin et al., "A novel and efficient synthesis of selenides", ARKIVOC, 2012, viii, 146-156)

Das Produkt konnte in einer guten Ausbeute erzielt werden, jedoch ist diese Syntheseroute sehr aufwendig, was es für einen großtechnischen Einsatz unattraktiv macht. Hierbei sind eine Vielzahl von Syntheseschritten notwendig, die zum Teil nicht unkritisch in ihrer Durchführung sind, insbesondere wenn man an eine Hochskalierung denkt und Maßstäbe verwendet, die in der Industrie üblich sind. Weiterhin fallen bei dieser Syntheseroute große Mengen an Abfallprodukten und Lösungsmittel an, die aufwändig entsorgt werden müssen, unter anderem auf Grund des Einsatzes von Brom.

Der Erfindung lag die Aufgabe zugrunde, ein Verfahren bereitzustellen, welches die in Zusammenhang mit dem Stand der Technik beschriebenen Nachteile nicht aufweist. Insbesondere soll ein Verfahren bereitgestellt werden, mit welchem Selenobiarylether in guter Ausbeute Hergestellt werden können. Das Verfahren sollte auch großtechnisch einsetzbar sein, und deshalb möglichst wenig Einzelschritte und Zwischenstufen aufweisen.

Gelöst wird die Aufgabe durch ein Verfahren nach Anspruch 1.

Verfahren zur Herstellung von Selenobiarylethern umfassend die Verfahrensschritte:
a) Zugabe eines ersten Arens zum Reaktionsgemisch,
b) Zugabe eines zweiten Arens zum Reaktionsgemisch,
c) Zugabe von Selendioxid zum Reaktionsgemisch,
d) Zugabe einer Säure mit einem pKs-Wert im Bereich von 0 bis 5 zum Reaktionsgemisch,
e) Einstellen der Reaktionstemperatur des Reaktionsgemisches, so dass das erste Aren und das zweite Aren zu einem Selenobiarylether umgesetzt werden.

Die Schritte a) bis d) können hierbei in beliebiger Reihenfolge durchgeführt werden. Dementsprechend liegt bei der Zugabe auch schon ein Reaktionsgemisch vor oder eben auch nicht, wie bei der ersten Zugabe in das Reaktionsgefäß.
Das Verfahren ist nicht auf die oben dargestellten Komponenten beschränkt. Weitere Bestandteile wie beispielsweise Lösungsmittel können ebenfalls in dem Reaktionsgemisch enthalten sein.

Verfügt die Säure über mehr als einen pKs-Wert, so ist der pKs₁-Wert heranzuziehen. Dieser muss im erfindungsgemäßen Fall im Bereich von 0 bis 5 liegen. Die Definition des pKs-Wertes ist dem Fachmann hinlänglich bekannt und kann der entsprechenden Fachliteratur entnommen werden.

Ein Vorteil gegenüber der im Stand der Technik beschriebenen Verfahren ist, dass hierbei nicht unter Feuchtigkeits- oder Sauerstoffausschluss gearbeitet werden muss. Dies stellt einen deutlichen Vorteil gegenüber anderen Syntheserouten dar. Diese Verfahren hebt sich in vorteilhafter Weise von den bestehenden mehrstufigen Syntheserouten abhebt.

Über den pKs-Wert kann die Reaktion in Richtung Selenobiarylether gelenkt werden, so dass die anfallenden Nebenprodukte reduziert werden. Dadurch, dass überwiegend das gewünschte Hauptprodukt gebildet wird und die Bildung von höhermolekularen Überoxidationsprodukten verringert wird, wird die Aufarbeitung deutlich vereinfacht.

Nicht umgesetzte Edukte sowie eingesetzte Lösungsmittel können destillativ zurückgewonnen und für weitere Reaktionen verwendet werden. Somit erfüllt das erfindungsgemäße Verfahren die Erfordernisse eines ökonomischen, großtechnischen Verfahrens.

Ferner wird im erfindungsgemäßen Verfahren Selendioxid verwendet. Selendioxid ist ein Abfallprodukt aus der Metallaufreinigung und Erzraffination. Somit wird in dem hier beanspruchten Verfahren ein Abfallprodukt aus anderen Prozessen erneut wertschöpfend eingesetzt. Dies ist insbesondere vor dem Hintergrund der Nachhaltigkeit von Prozessen ein wichtiges Thema.

In einer Variante des Verfahrens handelt es sich bei dem ersten Aren in Verfahrensschritt a) um eine Verbindung gemäß der allgemeinen Formel I: 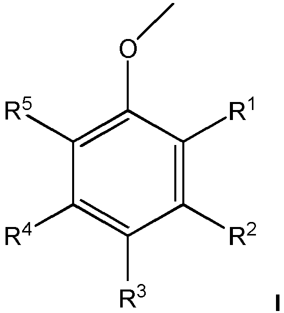
wobei R¹, R², R³, R⁴, R⁵ jeweils unabhängig voneinander ausgewählt sind aus:
   -H, -(C₁-C₁₂)-Alkyl, -O-(C₁-C₁₂)-Alkyl, -(C₆-C₂₀)-Aryl, -O-(C₆-C₂₀)-Aryl, -Halogen (wie Cl, F, Br, I), -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können,
und mindestens einer der Reste R¹, R², R³, R⁴, R⁵ gleich -H ist.

(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind aus:
(C₃-C₁₂)-Cycloalkyl, (C₃-C₁₂)-Heterocycloalkyl, (C₆-C₂0)-Aryl, Fluor, Chlor, Cyano, Formyl, Acyl oder Alkoxycarbonyl.

(C₆-C₂₀)-Aryl und O-(C₆-C₂₀)-Aryl können jeweils unsubstituiert oder durch einen oder mehrere gleiche oder verschiedene Reste substituiert sein, die ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂.

Im Rahmen der Erfindung umfasst der Ausdruck (C₁-C₁₂)-Alkyl geradkettige und verzweigte Alkylgruppen. Vorzugsweise handelt es sich dabei um unsubstituierte geradkettige oder verzweigte (C₁-C₈)-Alkylgruppen und ganz bevorzugt (C₁-C₆)-Alkylgruppen. Beispiele für (C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Dimethylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte (C₁-C₁₂)-Alkylgruppen und substituierte (C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt sind aus:
-(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

In einer Variante des Verfahrens sind R¹, R², R³, R⁴, R⁵ jeweils unabhängig voneinander ausgewählt aus:
-H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und mindestens einer der Reste R¹, R², R³, R⁴, R⁵ gleich -H ist.

In einer Variante des Verfahrens sind R¹, R², R³, R⁴, R⁵ jeweils unabhängig voneinander ausgewählt aus:
-H, -(C₁-C₁₂)-Alkyl, -O-(C₁-C₁₂)-Alkyl,
wobei die genannten Alkyl- und Arylgruppen substituiert sein können,
und mindestens einer der Reste R¹, R², R³, R⁴, R⁵ gleich -H ist.

In einer Variante des Verfahrens sind R¹, R², R³, R⁴, R⁵ jeweils unabhängig voneinander ausgewählt aus:
-H, -CH₃, -O-CH₃,
und mindestens einer der Reste R¹, R², R³, R⁴, R⁵ gleich -H ist.

In einer Variante des Verfahrens handelt es sich bei dem zweiten Aren in Verfahrensschritt b) um eine Verbindung gemäß der allgemeinen Formel II: 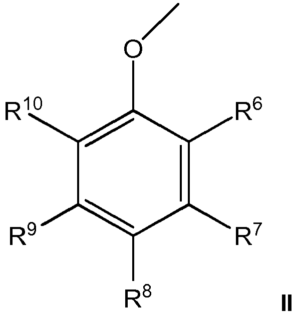
wobei R⁶, R⁷, R⁸, R⁹, R¹⁰ jeweils unabhängig voneinander ausgewählt sind aus:
   -H, -(C₁-C₁₂)-Alkyl, -O-(C₁-C₁₂)-Alkyl, -(C₆-C₂₀)-Aryl, -O-(C₆-C₂₀)-Aryl, -Halogen (wie Cl, F, Br, I), -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können,
und mindestens einer der Reste R⁶, R⁷, R⁸, R⁹, R¹⁰ gleich -H ist.

In einer Variante des Verfahrens sind R⁶, R⁷, R⁸, R⁹, R¹⁰ jeweils unabhängig voneinander ausgewählt aus:
-H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und mindestens einer der Reste R⁶, R⁷, R⁸, R⁹, R¹⁰ gleich -H ist.

In einer Variante des Verfahrens sind R⁶, R⁷, R⁸, R⁹, R¹⁰ jeweils unabhängig voneinander ausgewählt aus:
-H, -(C₁-C₁₂)-Alkyl, -O-(C₁-C₁₂)-Alkyl,
wobei die genannten Alkyl- und Arylgruppen substituiert sein können,
und mindestens einer der Reste R⁶, R⁷, R⁸, R⁹, R¹⁰ gleich -H ist.

In einer Variante des Verfahrens sind R⁶, R⁷, R⁸, R⁹, R¹⁰ jeweils unabhängig voneinander ausgewählt aus:
-H, -CH₃, -O-CH₃,
und mindestens einer der Reste R⁶, R⁷, R⁸, R⁹, R¹⁰ gleich -H ist.

In einer Variante des Verfahrens entspricht das erste Aren dem zweiten Aren.
Bei dieser Variante handelt es sich also um eine Homokupplung zweier gleicher Arene, welche über das Selen verknüpft werden.

In einer Variante des Verfahrens wird das Selendioxid in Verfahrensschritt c) in einem molaren Verhältnis bezogen auf die Summe des ersten und des zweiten Aren zugegeben, welches in einem Bereich von 0,25 bis 1,5 liegt.
Hierbei ist der Bereich von 0,25 bis 0,9 bevorzugt, und der Bereich von 0,4 bis 0,7 besonders bevorzugt.

In einer Variante des Verfahrens ist die Säure Essigsäure.

In einer Variante des Verfahrens wird die Säure im Verfahrensschritt d) als Lösungsmittel eingesetzt.

In einer Variante des Verfahrens wird das Reaktionsgemisch im Verfahrensschritt e) auf eine Temperatur in dem Bereich von 20 °C bis 100 °C eingestellt.
Hierbei ist der Bereich von 50 °C bis 100 °C bevorzugt, und der Bereich von 80 °C bis 90 °C besonders bevorzugt.

Im Folgenden wird die Erfindung anhand von Ausführungsbeispielen näher erläutert.

### Analytik

### NMR-Spektroskopie

Die NMR-spektroskopischen Untersuchungen wurden an Multikernresonanzspektrometern des Typs AC 300 oder AV II 400 der Firma *Bruker*, Analytische Messtechnik, Karlsruhe, durchgeführt. Als Lösungsmittel wurde CDCl₃ verwendet. Die ¹H- und ¹³C-Spektren wurden gemäß dem Restgehalt an nicht deuteriertem Lösungsmittel nach der *NMR Solvent Data Chart* der Fa. *Cambridge Isotopes Laboratories*, USA, kalibriert. Die Zuordnung der ¹H- und ¹³C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Bis(6-methyl-2,3,4-trimethoxyphenyl)selen

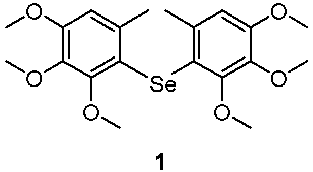

In einem 25 mL Rundkolben wurden 0.80 g 3,4,5-Trimethoxytoluol (4.3 mmol) in 6 mL Essigsäure gelöst, mit 0.27 g Selendioxid (2.4 mmol) versetzt und im 85 °C heißen Ölbad erwärmt. Nach 12 Tagen wurde das Reaktionsgemisch filtriert, das Filtrat mit Dichlormethan verdünnt und mit gesättigter Natriumchlorid-Lösung gewaschen. Die organische Phase wurde über Magnesiumsulfat getrocknet und das Lösungsmittel unter vermindertem Druck abdestilliert. Das Rohprodukt wurde mittels Säulenchromatographie auf gereinigt. Die Säulenlänge betrug 24 cm mit einem Durchmesser von 3 cm. Als Laufmittel wurde Cyclohexan/Ethylacetat im Verhältnis 9:1 verwendet.
Ausbeute: 0.399 g (0.9 mmol), 41%
GC: R(hart, HP-5) = 16.250 min
DC: R_{f}(CH:EE,2:1)=0.4
¹H-NMR: (400 MHz, CDCl3) δ [ppm] = 2.37 (s, 6H), 3.59 (s, 6H), 3.78 (s, 6H), 3.82 (s, 6H), 6.54 (s, 2H).
¹³C-NMR: (100 MHz, CDCl3) δ [ppm] = 23.52 56.00, 60.60, 60.86, 109.17, 118.21, 137.07, 140.44, 153.06, 154.19.
HRMS (ESI, pos.mode):m/z für [M+Na+]: berechnet: 465.0792 gefunden: 465.0780

### Bis(4,5-dimethoxy-2-methylphenyl)selen

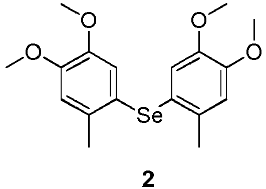

In einem 25 mL Rundkolben wurden 1.00 g 3,4-Dimethoxytoluol (6.5 mmol) in 9 mL Essigsäure gelöst, mit 0.40 g Selendioxid (3.6 mmol) versetzt und im 85 °C heißen Ölbad erwärmt. Nach 12 Tagen wurde das Reaktionsgemisch filtriert, das Filtrat mit Dichlormethan verdünnt und mit gesättigter Natriumchlorid-Lösung gewaschen. Die organische Phase wurde über Magnesiumsulfat getrocknet und das Lösungsmittel unter vermindertem Druck destilliert. Das Rohprodukt wurde mittels Säulenchromatographie auf gereinigt. Dabei wurde ein Säulenautomat der Firma BÜCHI-Labortechnik GmbH, Essen verwendet. Die Säulenlänge betrug 16 cm und der Durchmesser 6 cm. Als Eluent wurde Cyclohexan/Essigsäureethylester verwendet, wobei mit einem Essigsäureethylester Gradienten gearbeitet wurde: 0% (über 5 min), 1-5 % (über 5 min), 5-10% (über 8 min), 10-20% (8 min), 20-40% (10 min), 40-100% (10 min). Die Pumpgeschwindigkeit lag bei 100 mL/min.
Ausbeute: 0.637 g (1.6 mmol), 51%
GC: R(hart, HP-5) = 15.968 min
¹H-NMR: (400 MHz, CDCl3) δ [ppm] = 2.34 (s, 6H), 3.70 (s, 6H), 3.86 (s, 6H), 6.76 (s, 2H), 6.77 (s, 2H)
¹³C-NMR: (100 MHz, CDCl3) δ [ppm] = 21.96, 56.07, 56.16, 113.47, 116.51, 121.55, 132.50, 147.54, 148.70.
HRMS (ESI, pos.mode):m/z für [M+Na+]: berechnet: 405.0581 gefunden: 405.0484

Die beiden Verbindungen **1** und **2** konnten jeweils in sehr guten Ausbeuten synthetisiert werden. Somit wird die gestellte Aufgabe durch das erfindungsgemäße Verfahren gelöst.

## Patentansprüche

1. Verfahren zur Herstellung von Selenobiarylethern umfassend die Verfahrensschritte:
a) Zugabe eines ersten Arens zum Reaktionsgemisch,
b) Zugabe eines zweiten Arens zum Reaktionsgemisch,
c) Zugabe von Selendioxid zum Reaktionsgemisch,
d) Zugabe einer Säure mit einem pKs-Wert im Bereich von 0 bis 5 zum Reaktionsgemisch,
e) Einstellen der Reaktionstemperatur des Reaktionsgemisches, so dass das erste Aren und das zweite Aren zu einem Selenobiarylether umgesetzt werden.

2. Verfahren nach Anspruch 1,
wobei es sich bei dem ersten Aren in Verfahrensschritt a) um eine Verbindung gemäß der allgemeinen Formel **I** handelt: 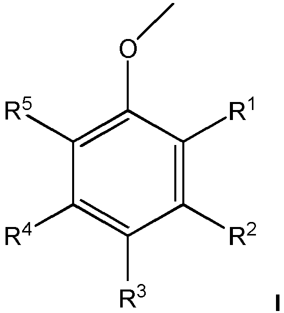
wobei R¹, R², R³, R⁴, R⁵ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können,
und mindestens einer der Reste R¹, R², R³, R⁴, R⁵ gleich -H ist.

3. Verfahren nach Anspruch 2,
wobei R¹, R², R³, R⁴, R⁵ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und mindestens einer der Reste R¹, R², R³, R⁴, R⁵ gleich -H ist.

4. Verfahren nach einem der Ansprüche 2 oder 3,
wobei R¹, R², R³, R⁴, R⁵ jeweils unabhängig voneinander ausgewählt sind aus:
-H, -(C₁-C₁₂)-Alkyl, -O-(C₁-C₁₂)-Alkyl,
wobei die genannten Alkylgruppen substituiert sein können,
und mindestens einer der Reste R¹, R², R³, R⁴, R⁵ gleich -H ist.

5. Verfahren nach einem der Ansprüche 1 bis 4,
wobei es sich bei dem zweiten Aren in Verfahrensschritt b) um eine Verbindung gemäß der allgemeinen Formel II handelt: 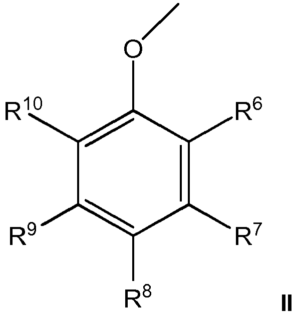
wobei R⁶, R⁷, R⁸, R⁹, R¹⁰ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können,
und mindestens einer der Reste R⁶, R⁷, R⁸, R⁹, R¹⁰ gleich -H ist.

6. Verfahren nach Anspruch 5,
wobei R⁶, R⁷, R⁸, R⁹, R¹⁰ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und mindestens einer der Reste R⁶, R⁷, R⁸, R⁹, R¹⁰ gleich -H ist.

7. Verfahren nach einem der Ansprüche 5 oder 6,
wobei R⁶, R⁷, R⁸, R⁹, R¹⁰ jeweils unabhängig voneinander ausgewählt sind aus:
-H, -(C₁-C₁₂)-Alkyl, -O-(C₁-C₁₂)-Alkyl,
wobei die genannten Alkyl- und Arylgruppen substituiert sein können,
und mindestens einer der Reste R⁶, R⁷, R⁸, R⁹, R¹⁰ gleich -H ist.

8. Verfahren nach einem der Ansprüche 1 bis 7,
wobei das erste Aren dem zweiten Aren entspricht.

9. Verfahren nach einem der Ansprüche 1 bis 8,
wobei das Selendioxid in Verfahrensschritt c) in einem molaren Verhältnis bezogen auf die Summe des ersten und des zweiten Aren zugegeben wird, welches in einem Bereich von 0,25 bis 1,5 liegt.

10. Verfahren nach einem der Ansprüche 1 bis 9,
wobei die Säure Essigsäure ist.

## Claims

1. Method for preparing selenobiaryl ethers comprising the method steps of:
a) adding a first arene to the reaction mixture,
b) adding a second arene to the reaction mixture,
c) adding selenium dioxide to the reaction mixture,
d) adding an acid having a pKa in the range from 0 to 5 to the reaction mixture,
e) adjusting the reaction temperature of the reaction mixture such that the first arene and the second arene are converted to a selenobiaryl ether.

2. Method according to Claim 1,
wherein the first arene in method step a) is a compound of the general formula **I:** 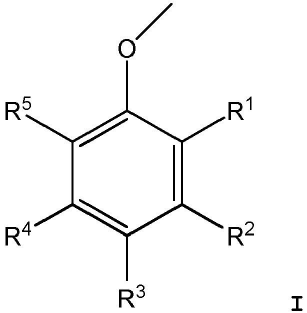
wherein R¹, R², R³, R⁴, R⁵ are each independently selected from:
-H, -(C₁-C₁₂)-alkyl, -O-(C₁-C₁₂)-alkyl, -(C₆-C₂₀)-aryl,-O-(C₆-C₂₀)-aryl, -halogen,
-OC=O-(C₁-C₁₂)-alkyl,
two adjacent radicals may additionally be joined to one another to form a condensed system,
where the alkyl and aryl groups mentioned may be substituted,
and at least one of the R¹, R², R³, R⁴, R⁵ radicals is-H.

3. Method according to Claim 2,
wherein R¹, R², R³, R⁴, R⁵ are each independently selected from:
-H, -(C₁-C₁₂)-alkyl, -O-(C₁-C₁₂)-alkyl, -(C₆-C₂₀)-aryl,-O-(C₆-C₂₀)-aryl,
where the alkyl and aryl groups mentioned may be substituted,
and at least one of the R¹, R², R³, R⁴, R⁵ radicals is-H.

4. Method according to either of Claims 2 and 3, wherein R¹, R², R³, R⁴, R⁵ are each independently selected from:
-H, -(C₁-C₁₂)-alkyl, -O-(C₁-C₁₂)-alkyl,
where the alkyl groups mentioned may be substituted, and at least one of the R¹, R², R³, R⁴, R⁵ radicals is-H.

5. Method according to any of Claims 1 to 4,
wherein the second arene in method step b) is a compound of the general formula **II:** 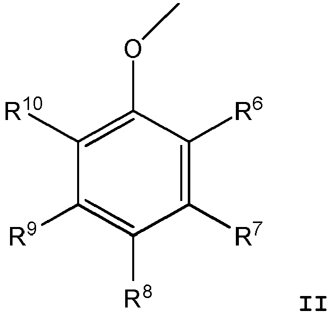
wherein R⁶, R⁷, R⁸, R⁹, R¹⁰ are each independently selected from:
-H, -(C₁-C₁₂)-alkyl, -O-(C₁-C₁₂)-alkyl, -(C₆-C₂₀)-aryl,-O-(C₆-C₂₀)-aryl, -halogen,
-OC=O-(C₁-C₁₂)-alkyl,
two adjacent radicals may additionally be joined to one another to form a condensed system,
where the alkyl and aryl groups mentioned may be substituted,
and at least one of the R⁶, R⁷, R⁸, R⁹, R¹⁰ radicals is-H.

6. Method according to Claim 5,
wherein R⁶, R⁷, R⁸, R⁹, R¹⁰ are each independently selected from:
-H, -(C₁-C₁₂)-alkyl, -O-(C₁-C₁₂)-alkyl, -(C₆-C₂₀)-aryl,-O-(C₆-C₂₀)-aryl,
where the alkyl and aryl groups mentioned may be substituted,
and at least one of the R⁶, R⁷, R⁸, R⁹, R¹⁰ radicals is-H.

7. Method according to either of Claims 5 and 6, wherein R⁶, R⁷, R⁸, R⁹, R¹⁰ are each independently selected from:
-H, -(C₁-C₁₂)-alkyl, -O-(C₁-C₁₂)-alkyl,
where the alkyl and aryl groups mentioned may be substituted,
and at least one of the R⁶, R⁷, R⁸, R⁹, R¹⁰ radicals is-H.

8. Method according to any of Claims 1 to 7,
wherein the first arene corresponds to the second arene.

9. Method according to any of Claims 1 to 8,
wherein the selenium dioxide is added in method step c) in a molar ratio based on the sum total of the first and second arenes within a range from 0.25 to 1.5.

10. Method according to any of Claims 1 to 9,
wherein the acid is acetic acid.

## Revendications

1. Procédé de fabrication d'éthers sélénobiaryliques comprenant les étapes de procédé suivantes :
a) l'ajout d'un premier arène au mélange réactionnel,
b) l'ajout d'un deuxième arène au mélange réactionnel,
c) l'ajout de dioxyde de sélénium au mélange réactionnel,
d) l'ajout d'un acide ayant une valeur de pKs dans la plage allant de 0 à 5 au mélange réactionnel,
e) l'ajustement de la température de réaction du mélange réactionnel pour que le premier arène et le deuxième arène réagissent pour former un éther sélénobiarylique.

2. Procédé selon la revendication 1, dans lequel le premier arène à l'étape de procédé a) est un composé selon la formule générale I : 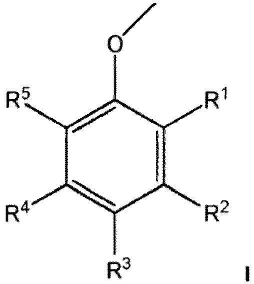
dans laquelle R¹, R², R³, R⁴, R⁵ sont chacun choisis indépendamment les uns des autres parmi :
-H, -alkyle en (C₁-C₁₂), -O-alkyle en (C₁-C₁₂), -aryle en (C₆-C₂₀), -O-aryle en (C₆-C₂₀), -halogène, -OC=O-alkyle en (C₁-C₁₂),
deux radicaux voisins pouvant en outre être reliés l'un à l'autre pour former un système condensé,
les groupes alkyle et aryle mentionnés pouvant être substitués,
et au moins un des radicaux R¹, R², R³, R⁴, R⁵ représentant -H.

3. Procédé selon la revendication 2, dans lequel R¹, R², R³, R⁴, R⁵ sont chacun choisis indépendamment les uns des autres parmi :
-H, -alkyle en (C₁-C₁₂), -O-alkyle en (C₁-C₁₂), -aryle en (C₆-C₂₀), -O-aryle en (C₆-C₂₀),
les groupes alkyle et aryle mentionnés pouvant être substitués,
et au moins un des radicaux R¹, R², R³, R⁴, R⁵ représentant -H.

4. Procédé selon l'une quelconque des revendications 2 ou 3, dans lequel R¹, R², R³, R⁴, R⁵ sont chacun choisis indépendamment les uns des autres parmi :
-H, -alkyle en (C₁-C₁₂), -O-alkyle en (C₁-C₁₂),
les groupes alkyle mentionnés pouvant être substitués, et au moins un des radicaux R¹, R², R³, R⁴, R⁵ représentant -H.

5. Procédé selon l'une quelconque des revendications 1 à 4, dans lequel le deuxième arène à l'étape de procédé b) est un composé selon la formule générale II : 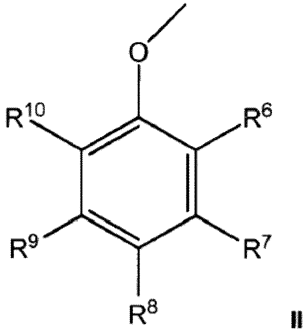
dans laquelle R⁶, R⁷, R⁸, R⁹, R¹⁰ sont chacun choisis indépendamment les uns des autres parmi :
-H, -alkyle en (C₁-C₁₂), -O-alkyle en (C₁-C₁₂), -aryle en (C₆-C₂₀), -O-aryle en (C₆-C₂₀), -halogène, -OC=O-alkyle en (C₁-C₁₂) ,
deux radicaux voisins pouvant en outre être reliés l'un à l'autre pour former un système condensé,
les groupes alkyle et aryle mentionnés pouvant être substitués,
et au moins un des radicaux R⁶, R⁷, R⁸, R⁹, R¹⁰ représentant -H.

6. Procédé selon la revendication 5, dans lequel R⁶, R⁷, R⁸, R⁹, R¹⁰ sont chacun choisis indépendamment les uns des autres parmi :
-H, -alkyle en (C₁-C₁₂), -O-alkyle en (C₁-C₁₂), -aryle en (C₆-C₂₀), -O-aryle en (C₆-C₂₀),
les groupes alkyle et aryle mentionnés pouvant être substitués,
et au moins un des radicaux R⁶, R⁷, R⁸, R⁹, R¹⁰ représentant -H.

7. Procédé selon l'une quelconque des revendications 5 ou 6, dans lequel R⁶, R⁷, R⁸, R⁹, R¹⁰ sont chacun choisis indépendamment les uns des autres parmi :
-H, -alkyle en (C₁-C₁₂), -O-alkyle en (C₁-C₁₂),
les groupes alkyle et aryle mentionnés pouvant être substitués,
et au moins un des radicaux R⁶, R⁷, R⁸, R⁹, R¹⁰ représentant -H.

8. Procédé selon l'une quelconque des revendications 1 à 7, dans lequel le premier arène correspond au deuxième arène.

9. Procédé selon l'une quelconque des revendications 1 à 8, dans lequel le dioxyde de sélénium à l'étape de procédé c) est ajouté en un rapport molaire par rapport à la somme du premier et du deuxième arène qui se situe dans une plage allant de 0,25 à 1,5.

10. Procédé selon l'une quelconque des revendications 1 à 9, dans lequel l'acide est l'acide acétique.
